# EUROPEAN PATENT APPLICATION

(11) **EP 1 972 336 A1**
(43) Date of publication of application: **24.09.2008**
(21) Application number: 07104410.1
(22) Date of filing: 19.03.2007
(51) Int. Cl.: A61K 9/16, A61K 31/192

(54) **Hot-melt micropellets**

(71) Applicant: LEK Pharmaceuticals d.d., 1526 Ljubljana (SI)
(72) Inventor: The designation of the inventor has not yet been filed
(74) Representative: Kröger, Bernd

(57) **Abstract**

The present invention provides a method of making solid micropellets of which at least 75% by weight have a diameter less than 500 µm comprising
(i) melting one or more binders; and
(ii) combining the one or more binders melted in step (i) with a pharmaceutically active ingredient to form solid micropellets.

Solid micropellets obtainable by this method are also provided. The invention has particular utility for improving the solubility of poorly water-soluble pharmaceutically active ingredients.

## Description

The present invention relates to a new method for making micropellets which comprise one or more binders and a pharmaceutically active ingredient, and the said micropellets obtained thereby. In particular, the invention has utility in increasing the solubility of poorly soluble pharmaceutically active ingredients.

Many techniques have been developed to improve the solubility of poorly water-soluble drugs. Some have focussed on the binders used in conjunction with the active ingredient. Binders which are amphiphilic in nature and dissolve readily in water have been shown to have particular utility in increasing a drug's solubility. Binders such as PEG, polyglycolyzed glycerides and polyoxypropylene-polyoxyethylene block copolymers may be heated with a poorly soluble drug for sufficient time to dissolve or disperse the active ingredient. The resulting composition may then be further processed into a pharmaceutical dosage form, with improved solubility of the active ingredient in comparison to the free active.

Other researchers have attempted to modify the physical form of the pharmaceutically active ingredient in the pharmaceutical composition to improve the active's release properties. A process known as melt granulation has been used to produce pellets of an active with controllable release properties.

Melt pelletisation is a further method which may be used for the preparation of pellets comprising a pharmaceutically active ingredient. Melt pelletisation is a process wherein materials of low melting point (~40-100 °C) are used as binders. The binders are typically either premixed with the powders and act as binders when heated above their melting point, or the binders are preheated, and sprayed whilst melted.

There remains a desire in the field of drug delivery to increase the solubility of poorly soluble pharmaceutically active ingredients whilst maintaining control over the rate of active ingredient release. In accordance with this unmet need, we provide in accordance with a first aspect of this invention a method of making solid micropellets of which at least 75% by weight have a diameter less than 500 µm comprising
(i) melting one or more binders;
(ii) combining the one or more binders melted in step (i) with a pharmaceutically active ingredient to form solid micropellets; and
(iii) allowing the binder to solidify.

Also provided, in a second aspect of this invention, are solid micropellets of which at least 75% by weight have a diameter less than 500 µm comprising a pharmaceutically active ingredient dispersed in a matrix of one or more binders. The matrix can be a continuous matrix.

We provide in a third aspect of the present invention a solid dosage form comprising the solid micropellets according to the second aspect of this invention, and one or more pharmaceutically acceptable excipients.

The solid and substantially spherical micropellets have been found to increase the solubility, in particular the solubility in aqueous acid solution, of pharmaceutically active ingredients, compared to conventional compositions used in drug delivery. Furthermore, with an appropriate choice of binder the bioavailability of the active can be precisely controlled. The small micropellet size, which is substantially smaller than pellets of pharmaceutically active ingredients in the prior art, is thought to contribute to the enhanced solubility and dissolution of the active ingredient.

Particular binders are preferred in the present invention. These are binders comprising polyglycolyzed glycerides and/or polyoxypropylene-polyoxyethylene block copolymers. Such binders have been shown to promote solid micropellet formation.

The present invention has particular utility in solid dosage forms wherein an analgesic active substance is incorporated and rapid onset of pain relief after administration is required. A rapid pharmacological effect can only be expected after quick dissolution of the active ingredient in the media of the gastro-intestinal tract (G.I.T.). Quick absorption is essential when non-steroidal anti-inflammatory drugs such as ketoprofen are administered, since the patient expects the painkiller to act quickly and a delay in the pharmacological effect can lead to the patient taking a second dose of the drug. If non-steroidal anti-inflammatory drugs remain in the G.I.T. for too long, the risk of side effects, such as bleeding, ulceration or perforation of the G.I.T increases. Unfortunately, non-steroidal anti-inflammatory drugs are not usually readily soluble in the media of the G.I.T. The present invention addresses this problem.

It will be appreciated that in a population of micropellets there will be a spread of particle diameters. The term micropellets, as used herein, refers to substantially spherical particles wherein at least 75% by weight of the micropellets have a diameter of less than 500 µm. Preferably, at least 85%, more preferably at least 90% and most preferably at least 95% of the micropellets by weight have a diameter less than 500 µm. In a preferred embodiment, at least 75%, more preferably at least 85%, 90% or 95% of the micropellets by weight have a diameter of less than 350 µm.

Preferably, the micropellets have a diameter of no smaller than 50 µm, more preferably no smaller than 100 µm. By this we mean that no more than 10%, preferably no more than 5% of the micropellets by weight have a diameter less than 50 µm or 100 µm.

The diameter of the micropellets may be measured by any suitable means known in the art. For instance, sieve analysis may be used.

The method according to the first aspect of this invention is a form of melt agglomeration. Melt agglomeration is a process by which solid fine particles are bound together into agglomerates, typically by agitation, in the presence of a molten binder. Dry agglomerates are obtained when the molten binder solidifies by cooling.

In the novel method, the pharmaceutically active ingredient may be added to the one or more binders prior to the step (i) of melting the one or more binders. This option is preferred when the emulsion method, or spray congealing are used to form the micropellets, as further detailed below. Preferably, however, the active ingredient is combined with the molten binder after melting of the binder in step (i) of the method, such as when a fluidised bed is used to form the micropellets. This contributes to achievement of a desirable particle size and shape distribution.

The method according to the first aspect of the present invention may be carried out in specialist equipment, although any means for heating the one or more binders and combining this with a pharmaceutically active ingredient to form said micropellets will suffice, provided the conditions are chosen to obtain the defined particle size. The specialist equipment may include rotating drums or pans, fluid-bed granulators, low shear mixers such as Z-blade and planetary mixers, and high shear mixers.

The meltable binder may be added as a molten liquid, or as dry powder or flakes. In the latter case, the binder may be heated by hot air or by a heating jacket to above the melting point of the binder.

Micropellet formation is typically promoted by mechanical agitation, kneading or layering. Further compounds (auxiliary ingredients), such as microcrystalline cellulose, may be combined with the binder and active ingredient to promote micropellet formation.

Preferably, in the first aspect of this invention, molten binder is sprayed onto solid pharmaceutically active ingredient. This has been shown to be particularly effective at producing micropellets, at least 75% by weight of which have a diameter less than 500 µm.

A particularly preferred apparatus for carrying out the method of the present invention comprises a fluidised bed together with rotary means, for instance comprising a fluid bed and a pelletiser, with an integrated rotor. A commercial example of such apparatus is a Glatt Particle Coater Granulator (GPCG) which comprises a fluid bed and a pelletiser, with an integrated rotor, and is particularly preferred. In the GPCG series, a modified GPCG I by Glatt GmbH may be used.

During pelletisation, the pharmaceutically active ingredient is mixed, optionally with one or more auxiliary ingredients (as detailed below) and the binder is sprayed onto it. One or more binders can be used and the centrifugal motion produces agglomerates, which are spheronised into micropellets.

Control of the conditions in the GPCG allows micropellets with a diameter of less than 500 µm to be produced. In the GPCG (or other suitable fluidised bed plus rotary means), typically the velocity of the inlet air is in the range 1-3ms⁻¹, most preferably 2ms⁻¹ (inlet air velocity is read from GPCG 1 control panel). The temperature of the inlet air is typically in the range 20-100 °C, preferably in the range 25-80°C, and most preferably in the range 30-60 °C. The chamber of the apparatus may have a starting temperature in the range 20-90 °C, preferably in the range 25-70°C, and most preferably in the range 30-50 °C.

The rotary plate which forms part of the rotor may be rough or smooth. Preferably, the rotary plate is smooth. Preferably, the rotary plate has a speed rotation when in use in the range 750-2000 RPM, most preferably 1000-1500 RPM, ideally around 1250 RPM. The pressure difference above and below the plate is typically 5-30 mbar, preferably 10-15 mbar.

The atomising pressure is typically 2-5 bar, most preferably around 3.5 bar, and the atomising air temperature is typically in the range 100-250°C, most preferably 130-200°C.

The binder is typically melted before being sprayed, and is therefore heated up to above its melting point. Typically, the binder is heated to 10-50 °C above its melting point, preferably to 30-40 °C above its melting point. Typically, the binder is heated to a temperature in the range 60-100 °C, more typically 70-90 °C, depending on its melting point.

The binder is sprayed into the apparatus chamber for sufficient time to enable micropellet formation. Typically, the binder will be sprayed for 2-20 minutes, more typically 3-10 minutes. Typically, 5-20 g/min of binder melt will enter the chamber via a peristaltic pump during the spraying time.

More specific preferred reaction parameters are given in the Examples.

The advantages of using a fluidised bed apparatus with rotation for the method of this invention include the fact that spherical pellets may be formed, precise quantities of pharmaceutically active ingredient may be incorporated, the active ingredient is evenly dispersed throughout the micropellet, the micropellets have a closed surface and high bulk density and that a narrow micropellet size distribution may be produced.

With regard to the binder, preferably this melts when heated to a temperature within the range 40-100°C, preferably in the range 40-70 °C. Using a suitable binder, a solid state solution or solid state dispersion of a poorly soluble pharmaceutically active ingredient may be obtained, which, in turn, provides a high degree of solubility to the active ingredient.

Binders which can be used for melt pelletisation include polyethylene glycols, waxes, fatty acids, mixtures of glycerides and esters of polyethylene glycols with fatty acids, fatty alcohols, and hydrogenated vegetable oils. However, particularly preferred binders in this invention are polyethylene glycol, polyglycolyzed glycerides and polyoxypropylene-polyoxyethylene copolymers, most preferably polyglycolyzed glycerides and polyoxypropylene-polyoxyethylene copolymers.

The binder used in the present invention typically has a hydrophilic/lipophilic balance value (HLB) greater than 10. Typically, the binder has a HLB value no greater than 30.

Polyglycolyzed glycerides may be prepared by an alcoholysis reaction of natural oils with polyoxyethylene glycols. In this invention the polyglycolyzed glycerides are preferably mixtures of monoesters, diesters and/or triesters of glycerides of long chain C₁₂ to C₁₈ fatty acids, and in polyethylene glycol mono- and/or diesters of long chain fatty acids. Suitable polyglycolyzed glycerides include those which are commercially known as Gelucires^{™}. Preferred polyglycolyzed glycerides, such as the Gelucire^{™} preparations, have melting points in the range from about 33°C to 64°C, as well as hydrophilic/lipophilic balance values (HLB) in the range from about 1 to about 14. The polyglycolyzed glycerides (such as Gelucires) of particular interest in the present invention have an HLB of above 10.

The first number in the nomenclature of a Gelucire denotes its melting point, whereas the second number provides the HLB value. The preferred Gelucires of the present invention are grades 44/14 and 50/13.

In general, the polyglycolyzed glycerides used as binders in the present invention have a HLB value in the range 1-18, preferably a HLB value greater than 10, most preferably in the range 10-14.

The polyoxypropylene-polyoxyethylene block copolymer is preferably of general formula:

HO(C₂H₄O)ₓ(C₃H₆O)_{y}(C₂H₄O)ₓH

wherein x is form 2 to 150 and y is from 15 to 70. Preferably, x from 12 to 141 and y is from 20 to 56.

Typically, the block copolymer has a molecular weight within the range 1000 to 16000.

The polyoxypropylene-polyoxyethylene block copolymer typically has a HLB value in the range 1-30, preferably more than 10, and most preferably more than 18.

Suitable polyoxypropylene-polyoxyethylene block copolymers are sold under the Pluronic and Lutrol trademarks. Preferred materials are block copolymers of polyoxyethylene and polyoxypropylene, generally having an average molecular weight from about 3,000 to about 15,000. The ethoxylated portion of the block copolymer generally constitutes from about 30 to about 80% by weight of the molecule. These include the Pluronic surfactants. Particularly good results are achievable with Pluronic F68, F108 and F127. Preferred polyoxypropylene-polyoxyethylene block copolymers (such as Pluronics) have an HLB above 10. For example, Pluronic F108 has an average molecular weight of 14,600, a polyoxyethylene content of about 80 weight % and an HLB value in excess of 24, and Pluronic F127 has an average molecular weight of 12,600, a polyoxyethylene content of about 70 weight % and an HLB value from 18 to 23.

Preferably, Lutrol F69 is used as a binder in this invention. A mixture of one or more binders may be used. Preferably, a combination of polyglycolyzed glyceride and a polyoxypropylene-polyoxyethylene copolymer is used. A combination of Lutrol F69 and Gelucire 44/14, for instance, is preferred.

An alternative method for making solid micropellets according to this invention is a spray congealing technique. One or more binders is mixed with a pharmaceutically active ingredient and heated to form a melt suspension. Typically, droplets of melt are sprayed from a nozzle into a current of cold gas. Solid micropellets of melt suspension are then formed after expulsion from the nozzle.

Alternatively, an emulsion may be formed wherein a water in oil emulsion is formed by heating, and then cooled to form solid micropellets. In this technique, a pharmaceutically active ingredient is dissolved or dispersed in a melt of hydrophilic binder. An outer oily phase, which may comprise oils of natural origin, is then heated to a temperature above the melting point of the binder and the melt of binder is dispersed within the outer oily phase, typically by means of a propeller mixer. The system is cooled in order to form solid micropellets. The oily phase is then decanted and the micropellets washed with solvent (for example, ether), in order to remove the surface residues of oily phase from the surface of the micropellets.

The pharmaceutically active ingredient may be any drug which necessitates controlled delivery. The active ingredient may be poorly soluble in water. By this we mean an active ingredient which has an intrinsic water solubility of less than 10.0 g/l, at pH7 and 25°C. Such active ingredients will be particularly benefited by the present invention.

Examples of pharmaceutically active ingredients are drugs belonging to the dihydropyridine class of compounds (e.g. nifedepine, felodipine, nicardipine), omeprazole, spironolactone, furosemide, terbutaline, riboflavine, gemfibrozil, indomethacin, ibuprofen, phenytoin, glyburide. In addition, any drug which has a water solubility of less than 10.0 g/l belonging to, for example, cardiovascular, cholesterol lowering, anti-hypertensive, antiepileptic, hormonal, hypoglycemic, antiviral, immunosuppressive, antihistaminic, nasal decongestant, antimicrobial, antiarrthrytic, analgesic, antimycobacterial, anticancer, diuretic, antifungal, antiparasitic, protein, peptide, CNS stimulants, CNS depressants, 5-HT inhibitors, anti-schizophrenia, anti-Alzheimer, antipsoriatic, steroidal, oligonucleotide, antiulcer, proton pump inhibitor, anti asthmatic, bronchodialators, thrombolytics or vitamin class of therapeutic agents, and any combinations thereof may be used in this composition in order to form solid state solutions and dispersions.

The solid micropellets of this invention have particular utility for increasing the solubility of pharmaceutically active ingredients in acidic media, typically acidic aqueous solution. In this embodiment, the active ingredient is typically poorly soluble in acidic aqueous solution. By this we mean that the intrinsic solubility is less than 10.0 g/l, at pH2 and 37°C. The solubility of ketoprofen is particularly increased using the micropellets of the present invention.

The invention is useful in any case where the pharmaceutically active ingredient is poorly soluble in aqueous solution at any of the values of physiological pH normally found within the G.I.T.. Thus, for instance, the solubility can be less than 10.0 g/l at the acidic pH (about 2) of the stomach or at the neutral to slightly alkaline pH of the ileum (pH7 to pH8), at 37°C. Preferably, the solubility is not more than 10.0 g/l at any pH in the range 2 to 8 at 37°C.

When the pharmaceutically active ingredient is ketoprofen, preferably the binder is a polyglycolyzed glyceride, a polyoxypropylene-polyoxyethylene copolymer, or a mixture of these.

In the micropellets of the present invention, the ratio of pharmaceutically active ingredient to binder is typically in the range 5:1 to 1:5, more typically in the range 5:1 to 1:1, most preferably in the range 3:1 to 2:1.

The amount of binder required is dependent upon whether the binder melt interacts with the pharmaceutically active ingredient. If the binder and active ingredient interact, then less binder is required compared to a case wherein there is no interaction.

The micropellets may additionally comprise one or more auxiliary ingredients selected from one or more disintegrants and/or lubricants and/or surfactants. These may be added to the binder and pharmaceutically active ingredient during the method according to the first aspect of this invention. Typically, the auxiliary ingredient is combined with the one or more binders and an active ingredient in step (ii) of the method according to the first aspect of this invention.

Disintegrants facilitate the breaking up of the orally disintegrating tablet in the gastro-intestinal tract. Suitable disintegrants may be selected from crosslinked sodium carboxymethylcellulose, crosslinked polyvinyl pyrrolidone, crosslinked carboxymethyl starch, other starch variants and microcrystalline cellulose, magnesium aluminium silicate and any combination thereof. Preferably, the disintegrant is crosslinked sodium carboxymethylcellulose.

Suitable surfactants include ionic surfactants, including anionic surfactants such as sodium lauryl sulfate, non-ionic surfactants such as different types of poloxamers, natural or synthesised lecithins and esters of sorbitan and fatty acids (such as Span), esters of polyoxyethylenesorbitan and fatty acids (such as Polysorbate 80) or cationic surfactants. Surfactants may also be referred to as wetting agents.

Examples of lubricants which can be used are magnesium stearate or calcium stearate, stearic acid, polyethylene glycols, sodium stearyl fumarate, silicon dioxide, hydrogenated vegetable oil, and behenic acid derivatives. Magnesium stearate and calcium stearate are particularly preferred.

The method of the present invention may further comprise a step of forming a solid dosage form from the sold micropellets, together with one or more pharmaceutically acceptable excipients. The solid dosage form is typically a tablet or a capsule.

The pharmaceutically acceptable excipients typically comprise a filler and/or a disintegrant and/or a lubricant/glidant.

The filler of the solid dosage form of the present invention may be selected from the group consisting of microcrystalline cellulose (MCC), modified forms of microcrystalline cellulose, lactose, sugars, different types of starch, modified forms of starch, mannitol, sorbitol and other polyols, dextrin, dextran and maltodextrin, calcium carbonate, calcium phosphate and/or hydrogen phosphate, sulphate and any combinations thereof. Fillers are added in concentrations 10-90 % to the total weight of the solid dosage form.

The disintegrant of the solid dosage form of the present invention may be selected from the group consisting of crosslinked sodium carboxymethylcellulose, crosslinked polyvinylpyrrolidone, crosslinked carboxymethyl starch, different types of starch and microcrystalline cellulose, magnesium aluminium silicate and any combinations thereof. Disintegrants are added in concentrations 2-20 % to the total mass of the solid dosage form.

The lubricant / glidant of the solid dosage form of the present invention may be selected from the group consisting of magnesium, calcium and zinc stearate, calcium behenate, sodium stearyl fumarate, talc, magnesium trisilicate, stearic acid, palmitic acid, carnauba wax, silicon dioxide and any combinations thereof. Lubricants and glidants are added in concentrations 0.1-10 % to the total mass of the solid dosage form.

The solid dosage forms of the present invention may be coated with a coating agent. Coating agents are typically used to mask an objectionable taste of a pharmaceutically active ingredient, to modify the release of the pharmaceutically active ingredient or to protect it from environmental influences.

The pharmaceutically active ingredient may have an objectionable taste or be irritating to the gastrointestinal tract. The coating composition should be selected accordingly. The coating composition may be pH dependent or pH independent. In the case of taste masking it is desirable to achieve the taste masking effect with the pH dependent protective coating. Typically, such a coating dissolves below pH 5 and remains intact in the pH range 5-14. As a result, in the oral cavity, which is normally a neutral environment with a pH around 7, the coating protects the pharmaceutically active ingredient and prevents the patient from being exposed to a bitter taste or irritating effect. In the acidic environment of the stomach the coating dissolves and the pharmaceutically active ingredient is released. As a rule, very thin coatings are necessary to protect the pharmaceutically active ingredient when a pH dependent coating is used.

The pH dependent coating used in the present invention may be selected from the group consisting of cationic polymers, more preferably polymethacrylates such as aminoalkyl methacrylate copolymers. The polymers marketed under the EUDRAGIT trademark are particularly preferred.

In the case of pH independent coatings, generally a thicker coating is necessary to protect the pharmaceutically active ingredient. pH independent coatings are typically used in patients with physiologically and/or pathologically elevated gastric pH. In such patients pH dependent coatings do not dissolve in the stomach. As a result, the pharmaceutically active ingredient is not absorbed from the gastro-intestinal tract and the pharmaceutically active ingredient's effect is lost.

The pH independent coating used in the present invention may be selected from the group consisting of ethyl cellulose, hydroxypropyl cellulose, hydroxyethyl cellulose, hydroxypropyl methyl cellulose, carboxymethylcellulose sodium, polyvinyl alcohol, polyvinyl pyrrolidone and any combinations thereof.

Further ingredients may be added to the coating composition to improve its characteristics. Protective film forming polymers can be mixed and processed together with other ingredients, such as polyethylene glycol, lactose, HPMC, HPC, microcrystalline cellulose or other water soluble or hydrophillic substances, to enhance permeability if required.

Other ingredients, such as plasticisers, glidants, pigments, solubilising agents may also be added to the coating composition to improve the suitability of the polymer dispersion for the coating. Micronised talc may be used as a glidant. Aromas may also be added to improve the taste masking effect of the coating composition.

The coating composition typically has a thickness of 2-30 µm, preferably 5-20 µm.

The mass of the solid dosage form is preferably no more than 500 mg, and most preferably in the range 200-500 mg.

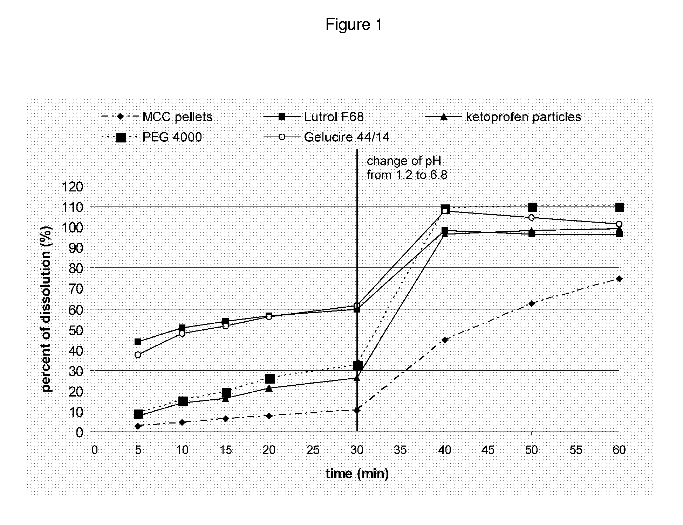

The invention will now be illustrated by the following Examples with reference to Figure 1, which shows the dissolution profile of ketoprofen released from the different types of micropellets, ketoprofen particles and ketoprofen loaded MCC micropellets.

### 1. Examples

Ketoprofen microparticles (125 - 355 µm) were produced using fluid bed hot-melt technology in combination with rotary processor. Microparticles were produced using one of the stated meltable binders: PEG 4000, Gelucire 44/14 or Lutrol F68. Used binders are amphiphilic in their nature and dissolve readily in water. Due to the stated properties of the binders and the intimate contact with the ketoprofen within the microparticle which emerges from method of production, improved rate of dissolution of microparticles produced with Gelucire 44/14 or Lutrol F68 has been found when compared to ketoprofen particles (Fig.1). Rate of dissolution has been improved in acidic media. This surprising behaviour has been assigned to solubilization effect of used binders. In case of microparticles produced with use of PEG 4000 no such effect could be observed. Nevertheless all prepared types of produced spherical microparticles exhibited faster dissolution rate when compared to microcrystalline cellulose matrix type pellets produced with extrusion spheronisation technology (Fig.1).

In Figure 1, the dissolution was carried out for 30 mins in 0.1 M HCl (pH = 1.2). The media was then switched to phosphate buffer (pH= 6.8).

### Detailed description of the micropellets preparation

Batches of 300 g of a mixture of ketoprofen and MCC were used in each experiment. Prior to micropellets preparation the apparatus (modified GPCG1, Glatt GmbH.) was heated to a set temperature, shut down and powder mixture was transferred into processing chamber. Binder material was molten in advance and thermostated to a set temperature during the process. Inlet air temperature was set to appropriate temperature for binder used and inlet air speed was set to 2 m/s in all experiments. The filter was shaken in alternate halves (i.e. one half of the filter was shaken at a time) at 3 second intervals for 5 seconds without interrupting the fluidization and binder spraying. Immediately after restarting the apparatus and adjusting process parameters binder melt was sprayed onto the circulating powders at a constant rate and rotor speed was set to 1250 RPM. Atomizing air pressure and atomizing air temperature were set in accordance with binder melt properties to ensure suitable binder droplet size. Inlet air temperature, starting chamber temperature and spray rate of binder melt were optimized for each binder separately. The experiments were terminated after required amount of binder was sprayed into processing chamber.

### Example 1: micropellets

| | |
|---|---|
| Ketoprofen | 150.0 g |
| Avicel PH105 | 150.0 g |
| PEG 4000 | 48.9 g |

Velocity of inlet air: 2 m/s
Temperature of inlet air: 57 °C
Starting chamber temperature: 49 °C
Type of rotary plate used: smooth
Rotation speed of the plate: 1250 RPM
Pressure difference below and above the plate: 12 mbar
Atomizing pressure: 2.5 bar
Atomizing air temperature: 180°C
Temperature of binder melt: 87°C
Rate of peristaltic pump: 15 RPM (16 g/min of binder melt)
Time of binder spraying: 3.05 min.

### Example 2: micropellets

| | |
|---|---|
| Ketoprofen | 150.0 g |
| Avicel PH105 | 150.0 g |
| Gelucire 44/14 | 48.9 g |

Velocity of inlet air: 2 m/s
Temperature of inlet air: 30°C
Starting chamber temperature: 32 °C
Type of rotary plate used: smooth
Rotation speed of the plate: 1250 RPM
Pressure difference below and above the plate: 12 mbar
Atomizing pressure: 3.0 bar
Atomizing air temperature: 175 °C
Temperature of binder melt: 70 °C
Rate of peristaltic pump: 9 RPM (7.26 g/min of binder melt)
Time of binder spraying: 6.74 min.

### Example 3: micropellets

| | |
|---|---|
| Ketoprofen | 150.0 g |
| Avicel PH105 | 150.0 g |
| Lutrol F68 | 48,90 g |

Velocity of inlet air: 2 m/s
Temperature of inlet air: 39°C
Starting chamber temperature: 36°C
Type of rotary plate used: smooth
Rotation speed of the plate: 1250 RPM
Pressure difference below and above the plate: 12 mbar
Atomizing pressure: 3.5 bar
Atomizing air temperature: 190°C
Temperature of binder melt: 95°C
Rate of peristaltic pump: 7 RPM (9.78 g/min of binder melt)
Time of binder spraying: 5.0 min.

## Claims

1. Method of making solid micropellets of which at least 75% by weight have a diameter less than 500 µm comprising
(i) melting one or more binders;
(ii) combining the one or more binders melted in step (i) with a pharmaceutically active ingredient; and
(iii) allowing the binder to solidify.

2. Method according to claim 1, where the pharmaceutically active ingredient is mixed with the one or more binders prior to the step (i) of melting the one or more binders.

3. Method according to claim 1, wherein the pharmaceutically active ingredient is dispersed in the one or more binders following the step (i) of melting the one or more binders.

4. Method according to any preceding claim, wherein in step (ii) the one or more binders and pharmaceutically active ingredient are subjected to mechanical agitation.

5. A method according to claim 1, wherein the one or more binders melted in step (i) are sprayed onto the pharmaceutically active ingredient in step (ii).

6. A method according to any of the preceding claims, wherein steps (i)-(iii) are carried out in a fluidised bed apparatus.

7. Method according to claim 6, wherein the fluidised bed apparatus comprises a rotary means which promotes the formation of the solid micropellets.

8. Method according to any preceding claim, wherein in step (i) the binders have a melting point in the range 40-100°C, preferably in the range 40-70°C.

9. Method according to any of the preceding claims, wherein the one or more binders are selected from polyethylene glycol, polyglycolyzed glycerides and polyoxypropylene-polyoxyethylene block copolymers.

10. Method according to claim 9, wherein the one or more binders are a polyglycolyzed glyceride or a polyoxypropylene-polyoxyethylene block copolymer.

11. Method according to any preceding claim, wherein the one or more binders have a hydrophilic/lipophilic balance value (HLB) in the range 10-14.

12. Method according to any preceding claim, wherein the pharmaceutically active ingredient is poorly soluble in aqueous solution at all pH values in the range 2 to 8 at 37°C.

13. Method according to claim 12, wherein the pharmaceutically active ingredient is poorly soluble in aqueous solution at pH2 and 37°C.

14. Method according to any preceding claim, wherein the pharmaceutically active ingredient is selected from a dihydropyridine, omeprazole, spironolactone, furosemide, terbutaline, riboflavine, gemfibrozil, indomethacin, ibuprofen, phenytoin, glyburide, or is a drug belonging to the cardiovascular, cholesterol lowering, anti-hypertensive, antiepileptic, hormonal, hypoglycemic, antiviral, immunosuppressive, antihistaminic, nasal decongestant, antimicrobial, antiarrthrytic, analgesic, antimycobacterial, anticancer, diuretic, antifungal, antiparasitic, protein, peptide, CNS stimulants, CNS depressants, 5-HT inhibitors, anti-schizophrenia, anti-Alzheimer, antipsoriatic, steroidal, oligonucleotide, antiulcer, proton pump inhibitor, anti asthmatic, bronchodialators, thrombolytics or vitamin class of therapeutic agents, and any combinations thereof.

15. Method according to claim 14, wherein the pharmaceutically active ingredient is an analgesic.

16. Method according to claim 15, wherein the analgesic is ketoprofen.

17. Method according to any of the preceding claims, wherein at least 75% by weight of the micropellets have a diameter less than 350 µm.

18. Method according to any of the preceding claims further comprising a step of forming a solid dosage form from the solid micropellets, together with one or more pharmaceutically acceptable excipients.

19. Method according to claim 18, wherein the solid dosage form is a tablet or capsule.

20. Solid micropellets of which at least 75% by weight have a diameter less than 500 µm comprising a pharmaceutically active ingredient dispersed in a matrix of one or more binders.

21. Solid micropellets according to claim 20, wherein the one or more binders have a melting point in the range 40-100°C, preferably in the range 40-70°C.

22. Solid micropellets according to claim 20 or claim 21, wherein the one or more binders are selected from polyethylene glycol, polyglycolyzed glycerides and polyoxypropylene-polyoxyethylene block copolymers.

23. Solid micropellets according to claim 22, wherein the one or more binders is a polyglycolyzed glyceride or a polyoxypropylene-polyoxyethylene block copolymer.

24. Solid micropellets according to any of claims 20-23, wherein the pharmaceutically active ingredient is poorly soluble in aqueous solution at all pH values in the range 2 to 8 at 37°C.

25. Solid micropellets according to claim 24, wherein the pharmaceutically active ingredient is poorly soluble in aqueous solution at pH2 and 37°C.

26. Solid micropellets according to any of claims 20-25, wherein the pharmaceutically active ingredient is selected from a dihydropyridine, omeprazole, spironolactone, furosemide, terbutaline, riboflavine, gemfibrozil, indomethacin, ibuprofen, phenytoin, glyburide, or is a drug belonging to the cardiovascular, cholesterol lowering, anti-hypertensive, antiepileptic, hormonal, hypoglycemic, antiviral, immunosuppressive, antihistaminic, nasal decongestant, antimicrobial, antiarrthrytic, analgesic, antimycobacterial, anticancer, diuretic, antifungal, antiparasitic, protein, peptide, CNS stimulants, CNS depressants, 5-HT inhibitors, anti-schizophrenia, anti-Alzheimer, antipsoriatic, steroidal, oligonucleotide, antiulcer, proton pump inhibitor, anti asthmatic, bronchodialators, thrombolytics or vitamin class of therapeutic agents, and any combinations thereof.

27. Solid micropellets according to claim 26, wherein the pharmaceutically active ingredient is an analgesic.

28. Solid micropellets according claim 27, wherein the pharmaceutically active ingredient is ketoprofen.

29. Solid micropellets according to any of claims 20 to 28, wherein at least 75% by weight of the micropellets have a diameter less than 350 µm.

30. A solid dosage form comprising solid micropellets according to any of claims 20 to 29, and one or more pharmaceutically acceptable excipients.

31. A solid dosage form according to claim 30, which is a tablet or capsule.

32. A solid dosage form according to claim 30 or 31, wherein the one or more pharmaceutically acceptable excipients comprise a filler and/or a disintegrant and/or a lubricant/glidant.
